# FASCICULE DE BREVET EUROPEEN

(11) **EP 1 172 094 B1**
(45) Date de publication et mention de la délivrance du brevet: **13.09.2006**
(21) Numéro de dépôt: 01401773.5
(22) Date de dépôt: 03.07.2001
(51) Int. Cl.: A61Q 19/08, A61K 8/63, A61K 8/97

(54) **Composition, notamment cosmétique, comprenant la DHEA et/ou un précurseur ou dérivé, et au moins un composé augmentant la synthèse des glycosaminoglycanes**
Mittel, insbesondere für Kosmetika, enthaltend DHEA und/oder Vorstufe oder Derivate davon, und mindestens eine Verbindung, die die Glykosaminoglykane-Synthese erhöht
Composition, particularly for cosmetics, comprising DHEA and/or precursor or derivative, and at least a compound which increases the synthesis of glycosaminoglycans

(30) Priorité: 13.07.2000 FR 0009221
(43) Date de publication de la demande: 16.01.2002
(73) Titulaire: L'ORÉAL, 75008 Paris (FR)
(72) Inventeur: Allec, Josiane, 06600 Antibes (FR); Cleren, Nathalie, 75012 Paris (FR)
(74) Mandataire: Renard, Emmanuelle

(56) Documents cités:
- EP-A- 0 908 183
- WO-A-96/40047
- WO-A-97/03676
- US-A- 5 989 568
- US-B- 4 542 129
- US-B- 5 019 391
- US-B- 5 961 981
- Ohio University -Algae taxonomy - Heterokontophyta, http://vis-pc.plantbio.ohiou.edu/algaeimag e/Heterokontophyta.htm

## Description

L'invention se rapporte à une composition comprenant, dans un milieu physiologiquement acceptable, la DHEA et/ou un précurseur ou dérivé chimique ou biologique de celle-ci, en association avec un extrait de Padina pavonica ainsi qu'à ses utilisations cosmétiques.

Il est connu que les signes du vieillissement cutané ont à la fois des causes chronologiques (vieillissement génétiquement programmé), et des facteurs additionnels aggravants tels que l'exposition au rayonnement UV et surtout les carences hormonales survenant lors de la ménopause. Ils se traduisent par des modifications de l'état de la peau généralement constatées par les femmes et confirmées par des études cliniques, à savoir :
- une perte de fermeté, de rebond, d'élasticité et de volume cutané et, à un âge plus avancé, un aspect papyracé de la peau,
- une sécheresse de la peau avec perte de souplesse, apparition de squames et de rugosités,
- une perte d'éclat du teint qui devient terne, et l'apparition de taches brunes.

Ces modifications sont essentiellement dues à un ralentissement et à un déséquilibre du fonctionnement cutané, qui se traduit par une « atrophie » de l'ensemble des assises de la peau. On observe ainsi une diminution de la qualité du derme (élastine, collagène, glycosaminoglycanes) et une perte de consistance de la matrice extracellulaire ; une diminution de l'épaisseur de l'épiderme (ralentissement du renouvellement cellulaire) ; un ralentissement de la production de lipides et des protéines de structure épidermique ; un déséquilibre de la desquamation ; et une réduction du contenu en eau de la peau.

Or, on cherche constamment des moyens de contrer, ou tout du moins de retarder, l'apparition de ces signes de vieillissement et de paraître ainsi plus jeune plus longtemps.

La Demanderesse a maintenant découvert que la qualité de la peau pouvait être préservée et améliorée en associant la DHEA et/ou ses précurseurs ou dérivés à un extrait de Padima pavonica.

La DHEA, ou déhydroépiandrostérone, est un stéroïde naturel produit essentiellement par les glandes corticosurrénales. Elle est connue pour sa capacité à promouvoir la kératinisation de l'épiderme (JP-07 196 467), ou encore dans le traitement des peaux sèches, en raison de son aptitude à augmenter la production endogène et la sécrétion de sébum et à renforcer l'effet barrière de la peau (US-4,496,556). Il a également été décrit dans le brevet US-5,843,932 l'utilisation de la DHEA pour remédier à l'atrophie du derme par inhibition de la perte de collagène et de tissu conjonctif. Il a enfin été proposé d'utiliser le sulfate de DHEA pour traiter différents signes du vieillissement tels que les rides, la perte d'éclat de la peau et le relâchement cutané (EP-0 723 775). On connaît également de la demande W09703676 des compositions contenant de la DHEA pour lutter contre les lipodystrophies ; et la demande EP 0 908 183 décrit l'utilisation de la DHEA ou de son sulfate pour augmenter la teneur en acide hyaluronique dans la peau.

Toutefois, à la connaissance de la Demanderesse, il n'a encore jamais été suggéré d'associer la DHEA et/ou ses précurseurs ou dérivés à un extrait de *Padina pavonica.*
Il est décrit dans le brevet US 5 961 981 l'utilisation d'extraits de plantes de la famille des Dictyotales (ex: *Padina pavonica)* dans des compositions pour stimuler la synthèse des glycosaminoglycanes, mais il n'est pas suggéré d'associer à ces extraits de la DHEA et/ou ses précurseurs ou dérivés.

L'invention a donc pour objet une composition comprenant, dans un milieu physiologiquement acceptable, la DHEA et/ou un précurseur ou dérivé chimique ou biologique de celle-ci, en association avec un extrait de *Padina pavonica.*

La DHEA a la formule (I) suivante : 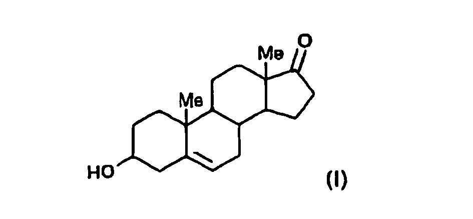 Elle est par exemple disponible auprès de la société AKZO NOBEL.

Par précurseurs de la DHEA, on entend ses précurseurs biologiques qui sont susceptibles de se transformer en DHEA au cours du métabolisme, ainsi que ses précurseurs chimiques qui peuvent se transformer en DHEA par réaction chimique exogène. Des exemples de précurseurs chimiques sont les sapogénines et leurs dérivés, tels que la diosgénine (ou spirost-5-èn-3-beta-ol), l'hécogénine, l'acétate d'hécogénine, le smilagénine et la sarsapogénine, ainsi que les extraits végétaux en contenant, en particulier le fenugrec et les extraits de Dioscorées telles que la racine d'igname sauvage ou Wild Yam, sans que cette liste soit limitative.
Selon une forme d'exécution préférée, l'analogue de DHEA est présent dans la composition selon l'invention sous forme d'un extrait d'igname sauvage, tel que celui commercialisé par la société ACTIVE ORGANICS sous la dénomination commerciale Actigen Y.
Par dérivés de la DHEA, on entend aussi bien ses dérivés biologiques que ses dérivés chimiques. Comme dérivés biologiques, on peut citer notamment le Δ5-androstène-3,17-diol et la Δ4-androstène-3,17-dione, sans que cette liste soit limitative. Comme dérivés chimiques, on peut citer notamment les sels de DHEA, en particulier les sels hydrosolubles, tels que le sulfate de DHEA. On peut citer également les esters, tels que les esters d'acides hydroxycarboxyliques et de DHEA décrits notamment dans US-5,736,537 ou les autres esters tels que le salicylate, l'acétate, le valérate (ou n-heptanoate) et l'énanthate de DHEA. On peut également citer les dérivés de DHEA (carbamates de DHEA, esters de 2-hydroxy malonate de DHEA et esters d'amino-acides de DHEA) décrits dans la demande FR 00/03846 au nom de la Demanderesse. Cette liste n'est évidemment pas limitative.
Les précurseurs et dérivés biologiques et chimiques de DHEA seront désignés ci-dessous par "analogues de DHEA".
La DHEA et/ou ses analogues peuvent être présents en une quantité allant de 0,0001 à 20% en poids, et de préférence de 0,001 à 5% en poids, par rapport au poids total de la composition.

D'extrait de *Padina pavonice* est une algue brune vivant dans les eaux peu profondes de la Méditerranée, disponible notamment auprès de la société ALBAN MULLER INTERNATIONAL sous la dénomination commerciale HPS3 :

L'extrait contenant de Padina pavonica représente par exemple de 0,0001 à 20%, et de préférence de 0,001 à 5%, du poids total de la composition selon l'invention.

Selon une forme d'exécution préférée de l'invention, la composition renferme en outre au moins un inhibiteur de métalloprotéinase, tel que le lycopène, les isoflavones, leurs dérivés ou les extraits végétaux en contenant, en particulier les extraits de soja (commercialisé par exemple par la société ICHIMARU PHARCOS sous la dénomination commerciale Flavostérone SB), de trèfle rouge, de lin, de kakkon ou de sauge. En variante ou en plus, la composition selon l'invention peut contenir un agent stimulant la croissance et/ou le métabolisme des fibroblastes, tel que des protéines ou polypeptides végétaux, extraits notamment du soja (par exemple un extrait de soja commercialisé par la société LSN sous la dénomination Eleseryl SH-VEG 8 ou commercialisé par la société SILAB sous la dénomination commerciale Raffermine), et/ou un agent favorisant la différenciation et/ou la croissance et/ou le métabolisme des kératinocytes, tel que le rétinol ou ses dérivés ou certains extraits végétaux tels qu'un extrait de lupin commercialisé par la société SILAB sous la dénomination commerciale Structurine, et/ou un agent stimulant la microcirculation cutanée tel qu'un extrait d'orange amère commercialisé par la société SILAB sous la dénomination commerciale Remoduline.

La concentration de ces agents additionnels facultatifs dans la composition selon l'invention peut varier de 0,0001 à 20% en poids et est de préférence comprise entre 0,001 et 5% en poids, par rapport au poids total de la composition.

La composition selon l'invention peut également contenir un agent favorisant la synthèse de collagène, tel que l'acide ascorbique ou ses dérivés, un agent exfoliant tel que les α- ou β-hydroxyacides, un agent protecteur ou filtre UV, un agent améliorant la barrière cutanée tel que des céramides, et/ou un agent hydratant tel que des polyols ou le pantothénate de calcium.

Selon un mode de réalisation particulièrement préféré de l'invention, la composition contient un extrait d'igname sauvage, un extrait de *Padina pavonica* et au moins un extrait de soja riche en isoflavones et/ou en protéines.

Selon un autre mode de réalisation, la composition selon l'invention contient en variante ou en plus un extrait de lupin riche en protéines.

La composition selon l'invention est destinée à une application topique sur la peau et renferme, de ce fait, un milieu physiologiquement acceptable, c'est-à-dire compatible avec la peau, ses phanères et les muqueuses.

La composition selon l'invention peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, en particulier sous forme de stick. Elle peut être utilisée comme produit de soin et/ou comme produit de maquillage pour la peau.

De façon connue, la composition de l'invention peut contenir également les adjuvants habituels dans le domaine cosmétique, tels que les gélifiants hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association d'actifs selon l'invention.

Lorsque la composition de l'invention est une émulsion, la proportion de la phase grasse peut aller de 2 à 80 % en poids, et de préférence de 3 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite) ou des beurres (beurre de karité).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition selon l'invention peut être utilisée à des fins cosmétiques, pour prévenir ou traiter les signes de vieillissement cutané, en particulier la perte de souplesse et/ou la sécheresse de la peau et/ou le relâchement cutané.

La présente invention a donc également pour objet un procédé cosmétique de prévention et/ou de traitement des signes de vieillissement cutané, comprenant l'administration par voie topique de la composition précédemment décrite.

L'invention sera mieux comprise, et ses avantages ressortiront mieux, à la lumière des exemples suivants, qui sont donnés à titre illustratif. Dans ces exemples, les quantités sont indiquées en pourcentage pondéral.

### EXEMPLES

### Exemple 1 : Emulsion sous forme de crème

| | | |
|---|---|---|
| Mélange d'alcool cétéarylique et de glucoside cétéarylique | 4 | % |
| Mélange de stéarate de glycéryle et stéarate de PEG-100 | 1 | % |
| Alcool cétylique | 0,5 | % |
| Benzoate d'alkyles en C₁₂₋₁₅ | 2 | % |
| Polyisobutène hydrogéné | 5 | % |
| Vaseline | 2 | % |
| Huiles végétales | 3,5 | % |
| Cire de silicone | 2 | % |
| Silicone volatile | 5 | % |
| Extrait de *Padina pavonica* | 1 | % |
| Extrait d'igname sauvage | 0,7 | % |
| Extrait de soja riche en isoflavones | 1 | % |
| PEG-20 | 1 | % |
| Alcool | 3 | % |
| Glycérol | 7 | % |
| Gélifiants | 1,5 | % |
| Filtres UVA et UVB | 12,5 | % |
| Neutralisant | 1,5 | % |
| Conservateurs | QS | |
| Eau | QSP 100 | % |

### Exemple 2 : Sérum anti-âge

| | | |
|---|---|---|
| Tetraoctanoate de pentaérythrityle | 1 ,4 | % |
| Hydroxyde de sodium | 0.7 | % |
| Acide citrique | 1,2 | % |
| Gélifiants | 2,5 | % |
| Huile de silicone | 3 | % |
| Extrait de *Padina Pavonica* | 1 | % |
| Extrait d'igname sauvage | 1 | % |
| Extrait de soja riche en protéines | 5 | % |
| Extrait de soja riche en isoflavones | 1 | % |
| Extrait de feuilles de sauge | 0,02 | % |
| Alcool | 5 | % |
| Glycérol | 3 | % |
| Hexylène glycol | 0.6 | % |
| Conservateurs | QS | |
| Eau | QSP 100 | % |

## Revendications

1. Composition comprenant, dans un milieu physiologiquement acceptable, la DHEA et/ou un précurseur ou dérivé de celle-ci, choisi parmi : les sels et esters de DHEA, la diosgénine, l'hécogénine, l'acétate d'hécogénine, la smilagénine, la sarsapogénine et les extraits de Dioscorées, en association avec un extrait de *Padina pavonica.*

2. Composition selon la revendication 1, **caractérisée en ce qu'**elle comprend de 0.0001 à 20% en poids dudit extrait de *Padina pavonica*, par rapport au poids total de la composition.

3. Composition selon la revendication 2, **caractérisée en ce qu'**elle comprend de 0,001 à 5% en poids dudit extrait de *Padina pavonica,* par rapport au poids total de la composition.

4. Composition selon l'une quelconque des revendications 1 à 3, **caractérisée en ce que** ledit extrait de Dioscorée est un extrait de racine d'igname sauvage.

5. Composition selon l'une quelconque des revendications précédentes, **caractérisée en ce qu'**elle est destinée à une application topique sur la peau.

6. Composition selon l'une quelconque des revendications précédentes, **caractérisée en ce que** la DHEA et/ou son précurseur ou dérivé choisi parmi : les sels et esters de DHEA, la diosgénine, l'hécogénine, l'acétate d'hécogénine, la smilagénine, la sarsapogénine et les extraits de Dioscorées sont présents en une quantité allant de 0,0001 à 20% en poids, par rapport au poids total de la composition.

7. Composition selon la revendication 6, **caractérisée en ce que** la DHEA et/ou son précurseur ou dérivé choisi parmi : les sels et esters de DHEA, la diosgénine, l'hécogénine, l'acétate d'hécogénine, la smilagénine, la sarsapogénine et les extraits de Dioscorées sont présents en une quantité allant de 0,001 à 5% en poids, par rapport au poids total de la composition.

8. Composition selon l'une quelconque des revendications précédentes, **caractérisée en ce qu'**elle renferme en outre au moins un inhibiteur de métalloprotéinase, un agent stimulant la croissance et/ou le métabolisme des fibroblastes, et/ou un agent favorisant la différenciation et/ou la croissance et/ou le métabolisme des kératinocytes, et/ou un agent stimulant la microcirculation cutanée.

9. Composition selon la revendication 8. **caractérisée en ce que** ledit inhibiteur de métalloprotéinase est choisi parmi un extrait de soja riche en isoflavones, un extrait de trèfle rouge, un extrait de lin, un extrait de kakkon, un extrait de sauge.

10. Composition selon la revendication 8, **caractérisée en ce que** ledit agent stimulant la croissance et/ou le métabolisme des fibroblastes est un extrait de soja riche en protéines.

11. Composition selon l'une quelconque des revendications précédentes, **caractérisée en ce qu'**elle renferme un extrait d'igname sauvage, un extrait de *Padina pavonica* et au moins un extrait de soja riche en isoflavones et/ou en protéines.

12. Procédé cosmétique de prévention et/ou de traitement des signes de vieillissement cutané, comprenant l'administration par voie topique de la composition selon l'une quelconque des revendications 1 à 11.

13. Utilisation cosmétique de la composition selon l'une quelconque des revendications 1 à 11 pour prévenir ou traiter les signes de vieillissement cutané.

14. Utilisation cosmétique de la composition selon l'une quelconque des revendications 1 à 11 pour prévenir ou traiter la perte de souplesse et/ou la sécheresse de la peau et/ou le relâchement cutané.

## Claims

1. Composition comprising, in a physiologically acceptable medium, DHEA and/or a precursor or derivative thereof, chosen from: DHEA salts and esters, diosgenin, hecogenin, hecogenin acetate, smilagenin, sarsapogenin and extracts of Dioscorea plants, in combination with an extract of *Padina pavonica.*

2. Composition according to Claim 1, **characterized in that** it comprises from 0.0001% to 20% by weight of the said extract of *Padina pavonica* relative to the total weight of the composition.

3. Composition according to Claim 2, **characterized in that** it comprises from 0.001% to 5% by weight of the said extract of *Padina pavonica* relative to the total weight of the composition.

4. Composition according to any one of Claims 1 to 3, **characterized in that** the said Dioscorea extract is an extract of the root of wild yam.

5. Composition according to any one of the preceding claims, **characterized in that** it is intended for topical application to the skin.

6. Composition according to any one of the preceding claims, **characterized in that** the DHEA and/or the precursor or derivative thereof chosen from: DHEA salts and esters, diosgenin, hecogenin, hecogenin acetate, smilagenin, sarsapogenin and extracts of Dioscorea plants are present in an amount ranging from 0.0001% to 20% by weight relative to the total weight of the composition.

7. Composition according to Claim 6, **characterized in that** the DHEA and/or the precursor or derivative thereof chosen from: DHEA salts and esters, diosgenin, hecogenin, hecogenin acetate, smilagenin, sarsapogenin and extracts of Dioscorea plants are present in an amount ranging from 0.001% to 5% by weight relative to the total weight of the composition.

8. Composition according to any one of the preceding claims, **characterized in that** it also contains at least one metalloprotease inhibitor, an agent for stimulating the growth and/or metabolism of fibroblasts, and/or an agent for promoting the differentiation and/or growth and/or metabolism of keratinocytes, and/or an agent for stimulating skin microcirculation.

9. Composition according to Claim 8, **characterized in that** the said metalloprotease inhibitor is chosen from an isoflavone-rich extract of soybean, an extract of red clover, an extract of flax, an extract of kakkon and an extract of sage.

10. Composition according to Claim 8, **characterized in that** the said agent for stimulating the growth and/or metabolism of fibroblasts is a protein-rich extract of soybean.

11. Composition according to any one of the preceding claims, **characterized in that** it contains an extract of wild yam, an extract of *Padina pavonica* and at least one isoflavone-rich and/or protein-rich extract of soybean.

12. Cosmetic process for preventing and/or treating the signs of ageing of the skin, comprising the topical administration of the composition according to any one of Claims 1 to 11.

13. Cosmetic use of the composition according to any one of Claims 1 to 11, to prevent or treat the signs of ageing of the skin.

14. Cosmetic use of the composition according to any one of Claims 1 to 11, to prevent or treat the loss of suppleness and/or the dryness of the skin and/or slackening of the skin.

## Patentansprüche

1. Zusammensetzung, die in einem physiologisch akzeptablen Medium DHEA und/oder einem Precursor oder ein Derivat von DHEA enthält, die ausgewählt sind unter: Salzen und Estern von DHEA, Diosgenin, Hecogenin, Hecogeninacetat, Smilagenin, Sarsapogenin und Discoreaceae-Extrakten, in Kombination mit einem Extrakt von *Padina pavonica.*

2. Zusammensetzung nach Anspruch 1, **dadurch gekennzeichnet, dass** sie 0,0001 bis 20 Gew.-% des Extraktes von *Padina pavoni*ca, bezogen auf das Gesamtgewicht der Zusammensetzung, enthält.

3. Zusammensetzung nach Anspruch 2, **dadurch gekennzeichnet, dass** sie 0,001 bis 5 Gew.-% des Extraktes von *Padina pavonica,* bezogen auf das Gesamtgewicht der Zusammensetzung, enthält.

4. Zusammensetzung nach einem der Ansprüche 1 bis 3, **dadurch gekennzeichnet, dass** der Dioscoreaceae-Extrakt ein Extrakt aus der Wurzel von Wildem Yams ist.

5. Zusammensetzung nach einem der vorhergehenden Ansprüche, **dadurch gekennzeichnet, dass** sie für eine topische Anwendung auf die Haut vorgesehen ist.

6. Zusammensetzung nach einem der vorhergehenden Ansprüche, **dadurch gekennzeichnet, dass** das DHEA und/oder seine Precursoren oder Derivate, die ausgewählt sind unter: Salzen und Estern von DHEA, Diosgenin, Hecogenin, Hecogeninacetat, Smilagenin, Sarsapogenin und Dioscoreaceae-Extrakten, in einem Mengenanteil von 0,0001 bis 20 Gew.-%, bezogen auf das Gesamtgewicht der Zusammensetzung, enthalten sind.

7. Zusammensetzung nach Anspruch 6, **dadurch gekennzeichnet, dass** das DHEA und/oder seine Precursoren oder Derivate, die ausgewählt sind unter: Salzen und Estern von DHEA, Diosgenin, Hecogenin, Hecogeninacetat, Smilagenin, Sarsapogenin und Dioscoreaceae-Extrakten, in einer Menge von 0,001 bis 5 Gew.-%, bezogen auf das Gesamtgewicht der Zusammensetzung, enthalten sind.

8. Zusammensetzung nach einem der vorhergehenden Ansprüche, **dadurch gekennzeichnet, dass** sie ferner mindestens einen Metalloproteinaseinhibitor, einen Stoff, der das Wachstum und/oder den Metabolismus von Fibroblasten stimuliert, und/oder einen Stoff, der die Differenzierung und/oder das Wachstum und/oder den Metabolismus von Keratinocyten fördert, und/oder einen Stoff, der die Mikrozirkulation der Haut stimuliert, enthält.

9. Zusammensetzung nach Anspruch 8, **dadurch gekennzeichnet, dass** der Metalloproteinaseinhibitor unter einem Sojaextrakt mit einem hohen Isoflavongehalt, einem Extrakt aus Rotklee, einem Extrakt aus Flachs, einem Extrakt aus Kakkon oder einem Extrakt aus Salbei ausgewählt ist.

10. Zusammensetzung nach Anspruch 8, **dadurch gekennzeichnet, dass** der Stoff, der das Wachstum und/oder den Metabolismus von Fibroblasten stimuliert, ein Sojaextrakt mit einem hohen Proteingehalt ist.

11. Zusammensetzung nach einem der vorhergehenden Ansprüche, **dadurch gekennzeichnet, dass** sie einen Extrakt aus Wildem Yams, einen Extrakt von *Padina pavonica* und mindestens einen Sojaextrakt mit einem hohen Isoflavongehalt und/oder hohen Proteingehalt enthält.

12. Kosmetisches Verfahren zur Vorbeugung und/oder zur Behandlung der Anzeichen der Hautalterung, das umfasst, auf topischem Wege die Zusammensetzung nach einem der Ansprüche 1 bis 11 aufzutragen.

13. Kosmetische Verwendung der Zusammensetzung nach einem der Ansprüche 1 bis 11 zur Vorbeugung oder Behandlung der Anzeichen der Hautalterung.

14. Kosmetische Verwendung der Zusammensetzung nach einem der Ansprüche 1 bis 11 zur Vorbeugung und/oder zur Behandlung des Verlustes der Geschmeidigkeit und/oder der Trockenheit der Haut und/oder des Schlafferwerdens der Haut.
